# EUROPEAN PATENT SPECIFICATION

(11) **EP 3 730 475 B1**
(45) Date of publication and mention of the grant of the patent: **26.07.2023**
(21) Application number: 19170770.2
(22) Date of filing: 24.04.2019
(51) Int. Cl.: C07C 45/29, C07C 49/407

(54) **METHOD FOR PREPARING L-MENTHONE**
VERFAHREN ZUR HERSTELLUNG VON L-MENTHON
PROCÉDÉ DE PRÉPARATION DE L-MENTHONE

(43) Date of publication of application: 28.10.2020
(73) Proprietor: BASF SE, 67056 Ludwigshafen am Rhein (DE)
(72) Inventor: SCHELWIES, Mathias, 67056 Ludwigshafen (DE); RENZ, Stephanie, 67056 Ludwigshafen (DE); THRUN, Frauke, 67056 Ludwigshafen (DE); PACIELLO, Rocco, 67056 Ludwigshafen (DE); GARLICHS, Florian, 68623 Lampertheim (DE); HEYDRICH, Gunnar, 67056 Ludwigshafen (DE); TELES, Joaquim Henrique, 67056 Ludwigshafen (DE); RUEDENAUER, Stefan, 67056 Ludwigshafen (DE); RIEDEL, Dominic, 67056 Ludwigshafen (DE)
(74) Representative: BASF IP Association

(56) References cited:
- YOUM J J Y ET AL: "Acceleration of bromide mediated benzoylperoxide oxidations of secondary alcohols in aqueous organic solvents", TETRAHEDRON LETTERS, ELSEVIER, AMSTERDAM, NL, vol. 49, no. 20, 12 May 2008 (2008-05-12), pages 3199-3203, XP022613618, ISSN: 0040-4039, DOI: 10.1016/J.TETLET.2008.03.098 [retrieved on 2008-03-23]
- SATO K ET AL: "A Practical Method for Alcohol Oxidation with Aqueous Hydrogen Peroxide under Organic Solvent- and Halide - Free Conditions", BULLETIN OF THE CHEMICAL SOCIETY OF JAPAN, CHEMICAL SOCIETY OF JAPAN, TOKYO, JP, vol. 72, 1 January 1999 (1999-01-01), pages 2287-2306, XP002266097, ISSN: 0009-2673, DOI: 10.1246/BCSJ.72.2287
- NOYORI R ET AL: "Green oxidation with aqueous hydrogen peroxide", CHEMICAL COMMUNICATIONS, ROYAL SOCIETY OF CHEMISTRY, UK, no. 16, 1 January 2003 (2003-01-01), pages 1977-1986, XP002533471, ISSN: 1359-7345, DOI: 10.1039/B303160H

## Description

The present invention relates to a method for preparing (-)-menthone from (+)-neomenthol.

(-)-Menthone is a stereoisomer of 2-Isopropyl-5-methylcyclohexanone, which has two asymmetric carbon centers and accordingly exists in the form of four stereoisomers, as depicted below:

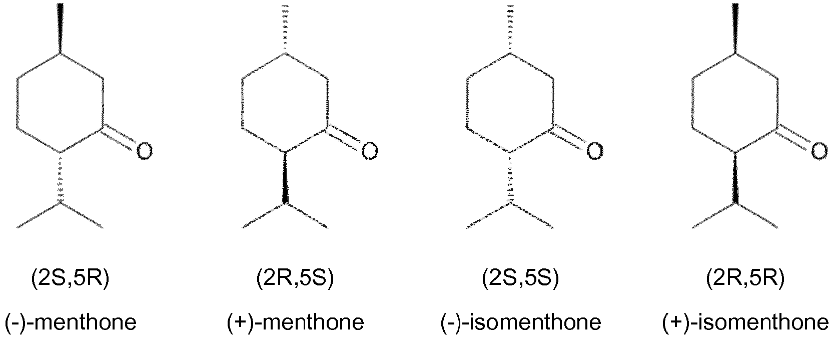

The (2S,5R) and (2R,5S) compounds are *trans*-isomers and are referred to as menthones. The (2S,5S) and (2R,5R) compounds are cis-isomers and are referred to as isomenthones.

Menthones and isomenthones are found in various essential oils, especially in oils of the *Mentha* genus. Menthones exhibit a characteristic minty odor, while isomenthones have a slightly musty odor (H. Surburg and J. Panten, Common Fragrance and Flavor Materials, 5th Edition (2006), Wiley-VCH, page 63 ff.). Menthones and isomenthones are commonly used for the preparation of synthetic peppermint oils and peppermint bases.

Industrial menthones are often mixtures of the above isomers in varying compositions. Menthones and isomenthones have a strong tendency to interconvert by reversible epimerization reaction via an enol intermediate and are, therefore, difficult to obtain in high purity. Specifically, (-)-menthone can interconvert to (+)-isomenthone and vice versa, and (+)-menthone can interconvert to (-)-isomenthone and vice versa.

Various methods for preparing menthone are known in literature. One common approach is directed to the oxidation of menthol. The term "menthol" relates to stereoisomers of 5-methyl-2-(propan-2-yl)cyclohexan-1-ol, which has three asymmetric carbon centers and accordingly exists in the form of eight stereoisomers, as depicted below:

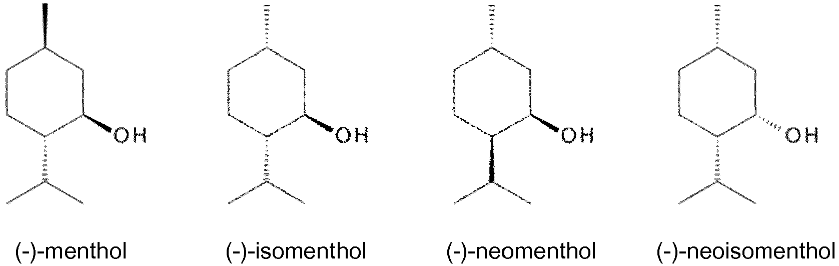

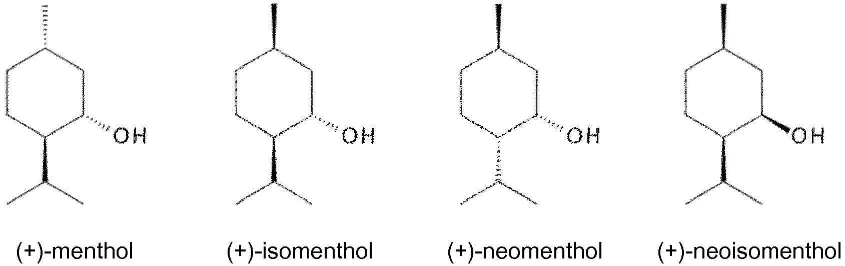

The industrially most important form, which is also the one occurring by far the most in nature, is (-)-menthol. In the production process of (-)-menthol, e.g., from (-)-isopulegol, a (+)-neomenthol stream may be obtained as a side product. Typically, the (+)-neomenthol stream is not further utilized, but rather disposed of.

Established methods for the oxidation of secondary alcohols such as menthol include the use of oxidizing agents based on toxic heavy metals (e.g., chromic acid or dichromate/sulfuric acid), or oxygen or air in the presence of a catalyst (e.g., N-oxyl compounds, EP 2 594 550 A1).

Noyori et al., J. Am. Chem. Soc. 1997, 119, 12386-12387, and Chem. Commun. 2003, 1977-1986, describe the oxidation of secondary alcohols with aqueous hydrogen peroxide in the presence of a tungsten catalyst and a phase-transfer catalyst. Tandon et al., Synlett 2005, (5), 872-873, describe further developments of the procedure described by Noyori et al., which include performing the reaction in tert-butanol. Both of these documents refer to menthol as a suitable substrate to be oxidized, but are silent on the stereoselectivity of the described methods.

K. Sato et al., Bull. Chem. Soc. Jpn. 1999, 72, 2287-2306 describes the preparation of (-)-menthone from (-)menthol by oxidising (-)menthol in the presence of a tungsten (VI) catalyst and a phase transfer catalyst using hydrogen peroxide as the oxidizing agent.

There is a need for providing an improved method for preparing (-)-menthone, where (-)-menthone can be obtained with high stereoselectivity. Further, the method should allow for preparing (-)-menthone with a high conversion of starting material and in high yield. Furthermore, the process allows for the utilization of a (+)-neomenthol stream obtained in a production process of (-)-menthol.

The aforementioned problems are solved by a method for preparing (-)-menthone by the process described hereinafter. The process comprises:
a) provision of a first mixture comprising (+)-neomenthol, a tungsten (VI) catalyst and a phase-transfer catalyst; and
b) addition of an aqueous hydrogen peroxide solution to the first mixture under reaction conditions to obtain a second mixture comprising (-)-menthone;
c) an aqueous work-up of the second mixture to obtain a purified (-)-menthone fraction, wherein the aqueous work-up comprises the addition of an aqueous solution of a formate salt to the second mixture.

Despite the typically occurring interconversion of (-)-menthone to (+)-isomenthone, it was found that the present method allows for the second mixture to comprise a high ratio of (-)-menthone to (+)-isomenthone, such as 90:10 or even higher. The present method accordingly allows for obtaining (-)-menthone with high stereoselectivity. Moreover, the process as described herein allows for the utilization of a (+)-neomenthol stream obtained in a production process of (-)-menthol.

It is believed that the addition of the aqueous hydrogen peroxide solution to the first mixture under reaction conditions allows for an especially mild oxidation conditions and high selectivity with regard to the formation of the desired (-)-menthone.

The term "reaction conditions" is understood to mean that during the addition of the aqueous hydrogen peroxide solution in step b), the temperature is suitable for inducing the oxidation of (+)-neomenthol to (-)-menthone. Suitable temperatures of the reaction mixture are in particular at least 65 °C, preferably at least 70 °C, especially at least 75 °C. The temperature of the reaction mixture will usually not exceed 90 °C, in particular not exceed 85 °C. In particular, the temperature of the reaction mixture is maintained in the range of 65 to 90 °C, more preferably in the range of 70 to 90 °C or in the range of 70 to 85 °C, especially in the range of 75 to 90 °C or 75 to 85 °C during the addition of the aqueous hydrogen peroxide solution to the first mixture.

For this, the first mixture is usually provided at a temperature of at least 60 °C, preferably at least 65 °C, in particular at least 70 °C. In particular, the temperature of the first mixture will not exceed 90 °C, in particular not exceed 85°C before the addition of the aqueous hydrogen peroxide solution is started. Thus the first mixture is preferably provided at a temperature in the range of 60 to 90 °C, more preferably in the range of 65 to 90 °C or in the range of 70 to 85 °C, especially in the range of 75 to 90 °C or 75 to 85 °C.

The pressure during the reaction is of minor importance. The reaction of step b) is usually performed at atmospheric pressure or higher, such as 1 to 1.5 bar, in particular 1.0 to 1.3 bar. All references herein to pressure refer to the absolute pressure unless noted otherwise. However, it is also possible to perform the reaction of step b) at a pressure below atmospheric pressure.

The aqueous hydrogen peroxide solution typically comprises hydrogen peroxide in a concentration of at least 15% by weight, preferably at least 25% by weight, and more preferably at least 35% by weight, based on the total weight of the aqueous hydrogen peroxide solution. The aqueous hydrogen peroxide solution typically comprises hydrogen peroxide in a concentration of at most 75% by weight, preferably at most 65% by weight, and most preferably at most 55% by weight, based on the total weight of the aqueous hydrogen peroxide solution. Preferably, the aqueous hydrogen peroxide solution comprises hydrogen peroxide in a concentration of 45 to 55% by weight, such as 50% by weight, based on the total weight of the aqueous hydrogen peroxide solution.

The amount of aqueous hydrogen peroxide solution added to the first mixture is generally chosen so that 0.8 to 1.4 moles of hydrogen peroxide are added per mole of (+)-neomenthol, preferably 0.85 to 1.3 moles, most preferably 0.9 to 1.2 moles, per mole of (+)-neomenthol.

Preferably, the aqueous hydrogen peroxide solution is added to the first mixture over an extended period of time. It is believed that this allows for avoiding high local concentrations of hydrogen peroxide. The hydrogen peroxide solution may be added incrementally or continuously. Frequently, the duration of the addition of the aqueous hydrogen peroxide solution may be in the range from 3 to 7 h, in particular in the range from 4 to 6 h. For example, the hydrogen peroxide solution may suitably be added at a rate of no more than 3.0 moles of hydrogen peroxide per mole of (+)-neomenthol per hour, preferably at a rate of no more than 2.0 moles of hydrogen peroxide per mole of (+)-neomenthol per hour.

The hydrogen peroxide solution is suitably added at a rate such that the resulting reaction heat can be efficiently removed from the reactor. In particular the resulting reaction heat should not exceed the possible rate of heat removal from the reactor. Apart from that, the addition rate of hydrogen peroxide solution should be chosen such that an accumulation of large amounts of unreacted hydrogen peroxide is avoided. For example, in case a reactor is used wherein the heat transfer rate is higher than the reaction rate, the aqueous hydrogen peroxide is suitably added at such a rate that at no point of time during the reaction more than 50% of the total amount of hydrogen peroxide is accumulated unreacted in the reaction mixture. The accumulation of unreacted hydrogen peroxide may be determined by reaction calorimetry, as known to the person skilled in the art.

The aqueous hydrogen peroxide solution may also be added to the first mixture over an extended period of time at a rate sufficient to carry out step b) at a temperature of at least 65 °C, in particular at least 70°C and especially at least 75 °C. The temperature is typically at most 90 °C, preferably at most 85 °C. It is understood that "carrying out step b)" at a given temperature refers to the time between the start of hydrogen peroxide addition and the end of the reaction. In particular, step b) is carried out at a temperature in the range of 65 to 90 °C, more preferably in the range of 70 to 85 °C or in the range of 75 to 85 °C. A skilled person understands that the rate of adding the hydrogen peroxide solution suitable to carry out step b) at a temperature of the given values depends on the total amount and composition of the reaction mixture.

In particular, the hydrogen peroxide solution is added at a rate of 1.0 to 3.0 moles of hydrogen peroxide per mole of (+)-neomenthol per hour, and step b) is carried out at a temperature in the range of 65 to 90 °C, more preferably in the range of 70 to 90 °C or in the range of 70 to 85 °C, especially in the range of 75 to 90 °C or 75 to 85 °C.

The first mixture comprises a tungsten (VI) catalyst. The term "tungsten (VI) catalyst" is understood to refer to any species comprising a tungsten atom in an oxidation state of +6 capable of catalyzing the oxidation reaction underlying the present method.

The tungsten (VI) catalyst is typically comprised in the first mixture in an amount of 0.01 to 3.5 mol-%, based on the amount of (+)-neomenthol in the first mixture, preferably 0.4 to 2.5 mol-%, more preferably 0.5 to 1.5 mol-% and most preferably 0.6 to 0.9 mol-%. The tungsten (VI) catalyst is preferably provided as an aqueous solution. This typically allows for better handling than adding the tungsten (VI) catalyst as a solid.

Suitable tungsten (VI) catalysts are for example orthotungstates, hydrates thereof, tungsten trioxide and hydrates thereof. Orthotungstate catalysts comprise a WO₄²⁻ anion and generally have the formula (M)₂WO₄, wherein M is a metal ion equivalent, e.g. Li⁺, Na⁺ or K⁺, or is an ammonium cation.

In one embodiment, the tungsten (VI) catalyst is selected from tungsten trioxide, i.e. WOs, and its monohydrate, WOs · H₂O (also referred to as tungstic acid, H₂WO₄), or mixtures thereof. When an aqueous hydrogen peroxide solution is added to the first mixture in step b) of the process, the active species is formed. Notably, this embodiment prolongs the method of the invention, as the *in-situ* generation of the tungsten (VI) catalyst requires additional reaction time. Further, the solubility of both tungsten trioxide and its monohydrate in water is significantly lower than, e.g. that of sodium tungstate dihydrate.

Therefore, preferably, the tungsten (VI) catalyst is an orthotungstate catalyst, i.e. a catalyst comprising a WO₄²⁻ anion. In a preferred embodiment, the tungsten (VI) catalyst is sodium tungstate, i.e. Na₂WO₄, or a hydrate thereof. Most preferably, the tungsten (VI) catalyst is sodium tungstate dihydrate, i.e. Na₂WO₄ · 2 H₂O. Sodium tungstate dihydrate advantageously has a high solubility in water.

The first mixture comprises a phase-transfer catalyst. A phase-transfer catalyst (PTC) facilitates the migration of a reactant from one phase into another phase. Typically, phase-transfer catalysts are quaternary ammonium salts, in particular in the form of halides, sulfates, hydrogen sulfates or organic sulfonates, but organic phosphonium salts such as hexadecyltributylphosphonium bromide may also be suitable.

Suitable phase-transfer catalysts are selected from the group consisting of compounds of the following formula (I)

R₄N⁺X⁻ (I)

wherein each R is independently selected from C₁-C₁₈-alkyl, C₆-C₁₈-aryl, C₅-C₁₈-heteroaryl and C₆-C₁₈-aryl-C₁-C₁₀-alkyl; and X- is an anion of a strong acid, such as halide, sulfate, methansulfonate, phosphate, hydrogen phosphate, dihydrogen phosphate or especially hydrogen sulfate.

Preferably, R is independently selected from C₁-C₁₈-alkyl, C₆-C₁₈-aryl and C₆-C₁₈-aryl-C₁-C₁₀-alkyl. More preferably, R is independently selected from C₁-C₁₈-alkyl and C₆-C₁₈-aryl-C₁-C₁₀-alkyl, especially C₁-C₁₈-alkyl.

The term "C₁-C₁₈-alkyl" is understood to refer to linear and branched acyclic hydrocarbons having a total of 1 to 18 carbon atoms. Suitable examples of C₁-C₁₈-alkyl include methyl, ethyl, propyl, butyl, pentyl, hexyl, octyl, decyl and dodecyl groups in both linear and branched form. Preferably, the C₁-C₁₈-alkyl is a C₁-C₁₂-alkyl.

The term "C₆-C₁₈-aryl" is understood to refer to monocyclic, bicyclic and tricyclic hydrocarbon ring systems having a total of 6 to 18 carbon atoms, wherein at least one ring in the system is aromatic and wherein each ring in the system contains three to seven ring members. Suitable examples of C₆-C₁₈-aryl include phenyl, biphenyl, indanyl, 1-naphthyl, 2-naphthyl and tetrahydronaphthyl. A preferred C₆-C₁₈-aryl is phenyl. Preferably, the C₆-C₁₈-aryl is a C₆-C₁₂-aryl.

The term "C₅-C₁₈-heteroaryl" is understood to refer to stable monocyclic and polycyclic aromatic hydrocarbons having a total of 5 to 18 carbon atoms and including at least one heteroatom ring member such as sulfur, oxygen, or nitrogen. Suitable examples of C₅-C₁₈-heteroaryl include pyridyl, pyrimidinyl, pyrazinyl, pyridazinyl, triazinyl, furyl, quinolyl, isoquinolyl, thienyl, imidazolyl, thiazolyl, indolyl, pyrroyl, oxazolyl, benzofuryl, benzothienyl, benzthiazolyl, isoxazolyl, pyrazolyl, triazolyl, tetrazolyl, indazolyl, 1,2,4-thiadiazolyl, isothiazolyl, purinyl, carbazolyl, benzimidazolyl, indolinyl, benzodioxolanyl and benzodioxane.

The term "C₆-C₁₈-aryl-C₁-C₁₀-alkyl" is understood to refer to a C₆-C₁₈-aryl moiety attached via a C₁-C₁₀-alkyl moiety to the remainder of the molecule. Suitable examples of C₆-C₁₈-aryl-C₁-C₁₀-alkyl include phenethyl and especially benzyl. Preferably, the Cs-C₁₈-aryl-C₁-C₁₀-alkyl is a C₆-C₁₂-aryl-C₁-C₆-alkyl.

A skilled person understands that by varying each of the moieties R, the properties of the phase-transfer catalyst, especially with regard to its solubility, can be optimized for the reaction.

In one embodiment, the first mixture comprises a quaternary ammonium halide of the formula (la)

R₄N⁺X⁻ (la)

wherein each R is independently selected from C₁-C₁₈-alkyl, C₆-C₁₈-aryl, C₅-C₁₈-heteroaryl and C₆-C₁₈-aryl-C₁-C₁₀-alkyl; and X- is a halide anion; and a strong acid, in particular sulfuric acid. Thereby a phase-transfer catalyst is obtained wherein the halide anion of the quaternary ammonium halide of the formula (la) is replaced with the corresponding anion of the strong acid, in particular with an HSO₄⁻ ion.

In formula (Ia), X- is a halide anion preferably selected from chloride, bromide and iodide, more preferably from chloride and bromide, especially chloride.

The quaternary ammonium halide of formula (Ia) may for example be selected from benzyltriethylammonium chloride, methyltributylammonium chloride and methyltrioctylammonium chloride. In a preferred embodiment, the quaternary ammonium halide of the formula (Ia) is methyltrioctylammonium chloride, i.e. MeOct₃N⁺Cl⁻, which is also known as "Aliquat 336" or "Starks' catalyst".

The term "strong acid" is understood to refer to any acid with a pkₐ value at 20 °C and atmospheric pressure (1 bar abs.) in water of less than 2, preferably less than 1. Where an acid has more than one pkₐ value due to having more than one acidic hydrogen moiety, it is considered to be a strong acid if its first, i.e. highest, pkₐ value at 20 °C and atmospheric pressure (1 bar abs.) is less than 2, preferably less than 1. The presence of a strong acid results in a pH of the reaction of below pH 7. It is believed that the low pH increases the effectivity of the oxidation of (+)-neomenthol to (-)-menthone.

The strong acid may be selected from organic acids such as sulfonic acids, e.g., p-toluenesulfonic acid or methanesulfonic acid. Preferably, the strong acid is an inorganic acid, more preferably a mineral acid. Suitable examples of strong acids include sulfuric acid, phosphoric acid and hydrochloric acid, especially sulfuric acid.

The strong acid may be present in the first mixture in an amount of 0.01 to 3.5 mol-%, based on the amount of (+)-neomenthol in the first mixture, preferably 0.4 to 2.5 mol-%, more preferably 0.5 to 1.5 mol-% and most preferably 0.6 to 0.9 mol-%. Preferably, the amount of the strong acid is chosen such that the pH of the resulting reaction mixture is below pH 7 (determined by a glass electrode at 20°C and 1 bar). Acid equivalent of the strong acid, i.e. molar amount of acidic protons provided by the strong acid is at least equimolar to the molar amount of the quaternary ammonium ions of the phase-transfer catalyst. Suitably, the strong acid is provided in the first mixture in at least equimolar amounts or in a slight excess over the quaternary ammonium halide of the formula (Ia), e.g., in a molar ratio of proton equivalents of the strong acid to the quaternary ammonium halide of the formula (Ia) of at least 1:1, e.g. 1:1 to 4:1 or 1:1 to 3:1 or 1:1 to 2.5 :1.

The phase-transfer catalyst may also be obtained by contacting the quaternary ammonium halide of the formula (Ia) with an anion exchanger, in particular with an anion exchanger charged with HSO₄⁻ anions. The quaternary ammonium halide of the formula (I) may be contacted with the anion exchanger either *in-situ* or prior to providing it to the first reaction mixture.

In preferred embodiments, the acid anion of the phase-transfer catalyst is acidic, e.g. hydrogensulfate. When the acid anion of the phase-transfer catalyst is not itself a strong acid, it is preferred that a strong acid is present in the first mixture regardless of the *in-situ* generation of phase-transfer catalyst, preferably a mineral acid such as sulfuric acid. In this case, additional strong acid may be added in accordance with the above embodiments.

In a particular preferred embodiment, the phase-transfer catalyst is a quaternary ammonium salt of the formula (Ib)

R₄N⁺HSO₄⁻ (Ib)

wherein each R is independently selected from C₁-C₁₈-alkyl, C₆-C₁₈-aryl, C₅-C₁₈-heteroaryl and C₆-C₁₈-aryl-C₁-C₁₀-alkyl. The embodiments described for the quaternary ammonium salts of the formula (I) with regard to R above also apply to the quaternary ammonium salt of the formula (Ib).

Suitable examples of such a phase-transfer catalyst include benzyltriethylammonium hydrogensulfate, methyltributylammonium hydrogensulfate, tetrabutylammonium hydrogensulfate and methyltrioctylammonium hydrogensulfate. Preferably, the phase-transfer catalyst is tetrabutylammonium hydrogensulfate or methyltrioctylammonium hydrogensulfate, and is in particular methyltrioctylammonium hydrogensulfate, i.e. MeOct₃N⁺HSO₄⁻.

The quaternary ammonium salt of the formula (la) may be provided as such or provided *in-situ* as described above by using sulfuric acid or an ion exchange resin charged with HSO₄⁻ anions.

In one embodiment, the (+)-neomenthol stream is provided as a neomenthol stream comprising (+)-neomenthol and at least one monoterpene alcohol, such as (+)-neoisomenthol, (-)-isopulegol or (-)-menthol or mixtures thereof. Such a stream may be obtained as a side product in the production of (-)-menthol, e.g., from (-)-isopulegol as the starting material. As the (+)-neomenthol stream obtained in the production of (-)-menthol is usually disposed of in current industrial processes, the present method allows for an added value to the production of (-)-menthol.

Typically, the (+)-neomenthol stream comprises (+)-neomenthol in an amount of at least 85% by weight, preferably at least 90% by weight, more preferably at least 95% by weight, based on the total weight of the (+)-neomenthol stream. As explained above, the (+)-neomenthol stream will usually contain at least one monoterpene alcohol different from (+)-neomenthol, in particular at least one of (+)-neoisomenthol, (-)-isopulegol and (-)-menthol. In such a (+)-neomenthol stream, the total amount of the monoterpene alcohols different from (+)-neomenthol is preferably in the range from 0.5 to 15% by weight, in particular in the range from 1 to 10% by weight and especially in the range from 2 to 5% by weight, based on the total weight of the (+)-neomenthol stream. The amount of (+)-neomenthol in such a (+)-neomenthol stream is consequently in the range from 85 to 99.5% by weight, in particular in the range from 90 to 99% by weight, especially in the range from 95 to 98% by weight, based on the total weight of the (+)-neomenthol stream.

In a particular embodiment of the invention, the (+)-neomenthol stream is provided as a neomenthol stream comprising (+)-neomenthol, (+)-neoisomenthol, (-)-isopulegol and (-)-menthol. In this particular embodiment, the (+)-neomenthol stream comprises (+)-neomenthol preferably in an amount from 80 to 99.5% by weight, in particular from 85 to 99% by weight, especially from 90 to 98% by weight, based on the total weight of (+)-neomenthol stream and monoterpene alcohols different from (+)-neomenthol in an amount from 0.5 to 20% by weight, in particular from 1 to 15% by weight and especially from 2 to 10% by weight, based on the total weight of the (+)-neomenthol stream, where the monoterpene alcohol different from (+)-neomenthol comprises (+)-neoisomenthol, (-)-isopulegol and (-)-menthol.

In one embodiment, the first mixture comprises an organic solvent. The term "organic solvent" is understood to refer to a non-aqueous organic solvent, which is different from the ingredients of the neomenthol stream. Examples of organic solvents include C₁-C₁₀ tertiary alcohols such as tert-butanol, tert-amylalcohol and 1-methylcyclohexanol; C₁-C₄ alkyl nitriles such as acetonitrile or propionitriles; aliphatic or cycloaliphatic ketones such as cyclohexanone; C₁-C₆ alkyl esters of C₁-C₆ alkanoic esters such as ethyl acetate and butyl acetate; ethers such as methyl-tert-butyl ether, tetrahydrofuran, tetrahydropyran, 2-methyltetrahydrofuran and methyl-cyclopentyl-ether; sulfones such as sulfolane; sulfoxides such as dimethyl sulfoxide (DMSO); di-(C₁-C₄-alkyl ether) glycols such as ethylene glycol dimethyl ether; di-(C₁-C₄-alkyl ether) polyalkylene glycols such as diethylene glycol dimethyl ether and triethylene glycol dimethyl ether; cyclic ureas such as N,N-dimethylimidazolidin-2-one and dimethylpropyleneurea (DMPU); and mixtures thereof.

The organic solvent is suitably chosen among compounds which are inert in the presence of the compounds of the first and second mixture and under the reaction conditions suitable for the present method. Suitable examples of the organic solvent for the present method include acetonitrile, tert-butanol and methyl-tert-butyl-ether.

It is understood that the term "organic solvent" does not comprise the compounds introduced by provision of the first mixture or by addition of the aqueous hydrogen peroxide according to steps a) and b) of the present method. For example, when the (+)-neomenthol stream is provided as a neomenthol stream comprising (+)-neomenthol and at least one of (+)-neoisomenthol, (-)-isopulegol and (-)-menthol, none of these compounds constitute "organic solvents". Likewise, water introduced via the aqueous hydrogen peroxide solution does not constitute an "organic solvent", in line with the definition of "organic solvent" above.

Advantageously, the method of the invention allows for providing the first and second mixture essentially free of an organic solvent, thus eliminating the need for removing and potentially recycling an organic solvent after completion of the reaction, which provides for a more economic process. The term "essentially free of" is understood to mean an amount of less than 10% by weight, preferably less than 7% by weight, more preferably less than 5% by weight, most preferably less than 3% by weight, such as less than 1% by weight or less than 0.1% by weight, based on the total weight of the first mixture. In a special embodiment, the first and second mixture does not comprise an organic solvent at all, i.e. the amount of organic solvent is 0.0% by weight, based on the total weight of the first mixture.

Steps a) and b) of the present method may be carried out in any conventional reactor suitable for the reaction conditions as described above as known to the skilled person. Thus, all reactors may be used which are generally suitable for reactions at the given temperature and the given pressure in the presence of reactive chemicals such as hydrogen peroxide. A suitable material in contact with the reaction mixture is vitreous enamel. Suitable standard reactors are named, for example, in K.D. Henkel, "Reactor Types and Their Industrial Applications", Wiley-VCH. Examples include stirred reactors, tanks, pumps, columns, and mixer settlers (phase separators).

It was found that, especially in the essential absence of an organic solvent, the second mixture obtained in step b) has a strong tendency to form an emulsion with the water present in the second mixture. This hampers phase separation and disadvantageously prolongs the work-up procedure which may be necessary to obtain a purified (-)-menthone fraction. As described above, the presence of an organic solvent brings with it its own disadvantages, e.g. a less economic process.

It was found that an aqueous work-up of the second mixture allows for an improved work-up by increasing the speed of phase separation.

Preferably, water is added in an amount sufficient so that an aqueous phase and an organic phase are formed during the aqueous work-up. It was found that the speed of phase separation is especially favorably high when the weight ratio of aqueous phase to organic phase is at least 1:1. Preferably, the aqueous work-up is carried out so that the weight ratio of aqueous phase to organic phase is at least 1.2:1, more preferably at least 1.5:1, such as 2:1. Of course, the amount of water used should not be too high from an economic standpoint. Accordingly, the weight ratio of aqueous phase to organic phase is preferably in the range of 1:1 to 3:1, more preferably 1.2:1 to 2.5:1, most preferably 1.5:1 to 2.2:1.

Further, it was found that the addition of a formate salt, to the second mixture during the aqueous work-up also allows for an improved work-up by increasing the speed of phase separation.

The aqueous work-up comprises the addition of an aqueous solution of a formate salt to the second mixture. Preferably, the formate salt is an alkali metal formate, more preferably sodium formate or potassium formate, especially sodium formate, which is preferably added as an aqueous solution once the reaction is complete.

The amount of formate salt is suitably sufficient to neutralize acid present in the second mixture. Preferably, the amount of formate added is in the range of 0.5 to 2 mole per acid neutralization equivalent. The term "neutralization equivalent" relates to the notional fraction of an acid molecule which is capable of providing a proton in the neutralization reaction in aqueous solution. For example, one molecule of H₂SO₄ corresponds to two neutralization equivalents, one molecule of H₃PO₄ to three neutralization equivalents.

Besides allowing for a faster phase separation, the addition of a formate salt to the second mixture in the course of the aqueous work-up neutralizes the strong acid optionally present. For example, if the first mixture comprises sulfuric acid, aqueous work-up comprising the addition of the formate salt to the second mixture neutralizes remaining H₂SO₄ and HSO₄⁻ ions in the aqueous and organic phases. Neutralization of the purified (-)-menthone fraction is advantageous so as to avoid acid-induced enolization, which can lead to an altered ratio of (-)-menthone to (+)-isomenthone in comparison to the ratio of the reaction mixture, in further processing steps such as distillation.

After the aqueous work-up or alternatively to working up, a (-)-menthone product may be separated, preferably by thermal separation such as distillation, from unreacted reactants, side components and, optionally, the solvent. The thermal separation takes place according to the methods known to a skilled person, preferably in an evaporator or in a distillation unit, comprising an evaporator and column(s) or a sequence of both. The distillation column(s) can be operated batchwise or continuously with column internals like trays, a structured packing or a random packing. The distillation is carried out at a maximum pressure of less than 50 mbar as measured at the column head, preferably less than 20 mbar. During start-up, the column is operated under total reflux and then the reflux ratio is adjusted to ≥ 2:1, preferably to a reflux ratio of ≥ 10:1. Unreacted (+)-neomenthol may be recycled back into the reaction. The separation of the product may also take place by crystallization. In one embodiment, an aqueous work-up of the second mixture is followed by a distillation step.

The high proportion of (-)-menthone in relation to (+)-isomenthone makes the reaction product particularly attractive, as the slightly musty odor attributed to isomenthones is reduced in comparison to mixtures comprising menthones and isomenthones with higher proportions of isomenthone.

The invention is illustrated in detail by the examples below.

### General Information

Gas chromatography analysis was performed according to the following method:
column: 50 m CP-WAX, FD: 1.2 µm, ID: 0.32 mm, 130 °C - 3° C/min - 150 °C - 20 min isotherm -10 °C/min 240 °C, constant pressure 0,73 bar, injector temperature: 250 °C, detector temperature: 280 °C. Diethylene glycol diethyl ether was used as internal standard.

### Example 1 (not according to the invention)

To a mixture of (+)-neomenthol (10 g, 64.0 mmol) and acetonitrile (6.4 mL) in a three-neck flask with reflux cooler, stir fish and internal thermometer were added tetrabutylammonium hydrogensulfate (0.53 g, 1.6 mmol, 2.5 mol-% relative to (+)-neomenthol) and sodium tungstate dihydrate (0.54 g, 1.6 mmol, 2.5 mol-% relative to (+)-neomenthol) under an argon atmosphere. The mixture was heated to 60 °C.

Subsequently, an aqueous solution of hydrogen peroxide (30% by weight, 6.0 mL, 58.8 mmol of H₂O₂, 0.92 molar equivalents relative to (+)-neomenthol) was added via syringe pump over the course of 9 h. Afterwards, the reaction mixture was stirred at 60 °C for 4 h.

Gas chromatography analysis showed that the mixture comprised (-)-menthone and (+)-isomenthone in a molar ratio of 96:4 (conversion of (+)-neomenthol: 83%).

### Example 2 (not according to the invention)

In a 100 mL three-neck flask with reflux cooler, dropping funnel, magnetic stirring bar and internal thermometer, 22.2 g of a (+)-neomenthol stream (90.9 wt.-% (129.1 mmol) of (+)-neomenthol and 9.1 wt.-% of a mixture of other menthol stereoisomers and L-menthon), sodium tungstate dihydrate (0.55 g, 1.6 mmol, 1.3 mol-% relative to (+)-neomenthol), methyltrioctylammonium chloride (0.67 g, 1.7 mmol, 1.3 mol-% relative to (+)-neomenthol) and sulfuric acid (98 wt.%, 0.17 g, 1.3 mol-% relative to (+)-neomenthol) were mixed under a nitrogen atmosphere. The mixture was heated to 70 °C.

Subsequently, an aqueous solution of hydrogen peroxide (50% by weight, 12 mL, 179.4 mmol of H₂O₂, 1.4 molar equivalents relative to (+)-neomenthol) was added via dropping funnel over the course of 75 min while the temperature of the reaction mixture did not exceed 80 °C. Afterwards, the reaction mixture was stirred at 70 °C for 3 h. The reaction mixture was subsequently cooled to room temperature and an aqueous solution of Na₂SO₃ (10 wt.-%, 30 mL) was added. The phases were separated and the organic phase was analyzed by gas chromatography.

Gas chromatography analysis showed that the organic phase comprised (-)-menthone and (+)-isomenthone in a molar ratio of 97:3 (conversion of (+)-neomenthol: 93%).

### Example 3

In a 750 mL double-jacketed glass vessel three-neck flask with reflux cooler, dropping funnel, glass impeller stirrer and internal thermometer, 400.0 g of a (+)-neomenthol containing stream of an L-menthol production (96.3 wt.-%, (2.46 mol) of (+)-neomenthol and 3.7 wt.-% of a mixture of other menthol stereoisomers and L-menthon), methyltrioctylammonium chloride (7.1 g, 17.6 mmol, 0.7 mol-% relative to (+)-neomenthol), sodium tungstate dihydrate (5.5 g, 17.6 mmol, 0.7 mol-% relative to (+)-neomenthol) and sulfuric acid (98 wt.%, 1.79 g, 17.6 mol-% relative to (+)-neomenthol) were mixed under a nitrogen atmosphere. The mixture was heated to 70 °C.

Subsequently, an aqueous solution of hydrogen peroxide (50% by weight, 196 g, 2.88 mol of H₂O₂, 1.15 molar equivalents relative to (+)-neomenthol) was added via dropping funnel over the course of 3 h while the temperature of the reaction mixture did not exceed 80 °C. Afterwards, the reaction mixture was stirred at 70 °C for 3 h. The reaction mixture was subsequently cooled to room temperature and stirred for 12 h. Afterwards, an aqueous solution of sodium formate (33 wt.-%, 90 g) was added. The phases were separated and the organic phase was extracted with an aqueous solution of sodium chloride (4 wt.-%, 235 g). The organic phase was analyzed by gas chromatography.

Gas chromatography analysis showed that the organic phase comprised (-)-menthone and (+)-isomenthone in a molar ratio of 96:4 (conversion of (+)-neomenthol: 99%).

## Claims

1. A method for preparing (-)-menthone which comprises:
a) provision of a first mixture comprising (+)-neomenthol, a tungsten (VI) catalyst and a phase-transfer catalyst; and
b) addition of an aqueous hydrogen peroxide solution to the first mixture under reaction conditions to obtain a second mixture comprising (-)-menthone;
c) an aqueous work-up of the second mixture to obtain a purified (-)-menthone fraction,
wherein the aqueous work-up comprises the addition of an aqueous solution of a formate salt to the second mixture.

2. The method according to claim 1, wherein (+)-neomenthol is provided as a neomenthol stream comprising (+)-neomenthol and at least one of (+)-neoisomenthol, (-)-isopulegol and (-)-menthol.

3. The method according to claim 2, wherein the neomenthol stream comprises (+)-neomenthol in an amount from 85 to 99% by weight, based on the total weight of the neomenthol stream.

4. The method according to any of the preceding claims, wherein during the addition of the aqueous hydrogen peroxide solution, a temperature of the reaction mixture in the range of 55 to 90 °C is maintained.

5. The method according to any of the preceding claims, wherein the tungsten (VI) catalyst is an orthotungstate catalyst.

6. The method according to claim 5, wherein the tungsten (VI) catalyst is sodium tungstate or a hydrate thereof.

7. The method according to any one of the preceding claims, wherein the phase-transfer catalyst is a compound of the formula (I)
R₄N⁺X⁻ (I)
wherein each R is independently selected from C₁-C₁₈-alkyl, C₆-C₁₈-aryl, C₅-C₁₈-heteroaryl and C₆-C₁₈-aryl-C₁-C₁₀-alkyl; and X- is an anion.

8. The method according to claim 7, wherein the first mixture comprises a compound of formula I, wherein X- is a halide anion, and a strong acid, in particular sulfuric acid.

9. The method according to claim 8, wherein the compound of formula (I) is methyltrioctylammonium chloride.

10. The method according to claim 7, wherein the phase-transfer catalyst contained in the first mixture is a quaternary ammonium salt of the formula (Ib)
R₄N⁺HSO₄⁻ (Ib),
wherein each R is independently selected from C₁-C₁₈-alkyl, C₆-C₁₈-aryl, C₅-C₁₈-heteroaryl and C₆-C₁₈-aryl-C₁-C₁₀-alkyl.

11. The method according to any of the preceding claims, wherein the first mixture and second mixture are essentially free of an organic solvent.

12. The method according to any one of the preceding claims, wherein water is added in an amount sufficient such that an aqueous phase and an organic phase are formed during the aqueous work-up.

13. The method according to claim 12, wherein the weight ratio of added water to organic phase is at least 1:1, in particular wherein the weight ratio of added water to organic phase is in the range of 1:1 to 3:1.

14. The method according to any one of the preceding claims, wherein the aqueous work-up comprises the addition of an aqueous solution of an alkali metal formate.

## Patentansprüche

1. Verfahren zur Herstellung von (-)-Menthon, das Folgendes umfasst:
a) Bereitstellung einer ersten Mischung, die (+)-Neomenthol, einen Wolfram(VI)-Katalysator und einen Phasentransferkatalysator umfasst; und
b) Zugabe einer wässrigen Wasserstoffperoxidlösung zu der ersten Mischung unter Reaktionsbedingungen zum Erhalt einer zweiten Mischung, die (-)-Menthon umfasst;
c) wässrige Aufarbeitung der zweiten Mischung zum Erhalt einer gereinigten (-)-Menthonfraktion,
wobei die wässrige Aufarbeitung die Zugabe einer wässrigen Lösung eines Formiatsalzes zu der zweiten Mischung umfasst.

2. Verfahren nach Anspruch 1, wobei (+)-Neomenthol als Neomentholstrom, der (+)-Neomenthol und mindestens eines von (+)-Neoisomenthol, (-)-Isopulegol und (-)-Menthol umfasst, bereitgestellt wird.

3. Verfahren nach Anspruch 2, wobei der Neomentholstrom (+)-Neomenthol in einer Menge von 85 bis 99 Gew.-%, bezogen auf das Gesamtgewicht des Neomentholstroms, umfasst.

4. Verfahren nach einem der vorhergehenden Ansprüche, bei dem während der Zugabe der wässrigen Wasserstoffperoxidlösung eine Temperatur der Reaktionsmischung im Bereich von 55 bis 90 °C beibehalten wird.

5. Verfahren nach einem der vorhergehenden Ansprüche, wobei es sich bei dem Wolfram(VI)-Katalysator um einen Orthowolframat-Katalysator handelt.

6. Verfahren nach Anspruch 5, wobei es sich bei dem Wolfram(VI)-Katalysator um Natriumwolframat oder ein Hydrat davon handelt.

7. Verfahren nach einem der vorhergehenden Ansprüche, wobei es sich bei dem Phasentransferkatalysator um eine Verbindung der Formel (I) handelt:
R₄N⁺X⁻ (I)
wobei R jeweils unabhängig aus C₁-C₁₈-Alkyl, C₆-C₁₈-Aryl, C₅-C₁₈-Heteroaryl und C₆-C₁₈-Aryl-C₁-C₁₀-alkyl ausgewählt ist und X⁻ ein Anion ist.

8. Verfahren nach Anspruch 7, wobei die erste Mischung eine Verbindung der Formel I, in der X⁻ ein Halogenid-Anion ist, und eine starke Säure, insbesondere Schwefelsäure, umfasst.

9. Verfahren nach Anspruch 8, wobei es sich bei der Verbindung der Formel (I) um Methyltrioctylammoniumchlorid handelt.

10. Verfahren nach Anspruch 7, wobei es sich bei dem in der ersten Mischung enthaltenen Phasentransferkatalysator um ein quartäres Ammoniumsalz der Formel (Ib) handelt:
R₄N⁺HSO₄⁻ (Ib)
wobei R jeweils unabhängig aus C₁-C₁₈-Alkyl, C₆-C₁₈-Aryl, C₅-C₁₈-Heteroaryl und C₆-C₁₈-Aryl-C₁-C₁₀-alkyl ausgewählt ist.

11. Verfahren nach einem der vorhergehenden Ansprüche, wobei die erste Mischung und die zweite Mischung im Wesentlichen frei von organischem Lösungsmittel sind.

12. Verfahren nach einem der vorhergehenden Ansprüche, wobei Wasser in einer solchen Menge zugegeben wird, dass sich während der wässrigen Aufarbeitung eine wässrige Phase und eine organische Phase bilden.

13. Verfahren nach Anspruch 12, wobei das Gewichtsverhältnis von zugegebenem Wasser zu organischer Phase mindestens 1:1 beträgt, insbesondere wobei das Gewichtsverhältnis von zugegebenem Wasser zu organischer Phase im Bereich von 1:1 bis 3:1 liegt.

14. Verfahren nach einem der vorhergehenden Ansprüche, wobei die wässrige Aufarbeitung die Zugabe einer wässrigen Lösung eines Alkalimetallformiats umfasst.

## Revendications

1. Procédé de préparation de (-)-menthone qui comprend :
a) la fourniture d'un premier mélange comprenant du (+)-néomenthol, un catalyseur à base de tungstène (VI) et un catalyseur de transfert de phase ; et
b) l'ajout d'une solution aqueuse de peroxyde d'hydrogène au premier mélange dans des conditions de réaction pour obtenir un deuxième mélange comprenant de la (-)-menthone ;
c) un traitement aqueux du deuxième mélange pour obtenir une fraction de (-)-menthone purifiée,
dans lequel le traitement aqueux comprend l'ajout d'une solution aqueuse d'un sel de formiate au deuxième mélange.

2. Procédé selon la revendication 1, dans lequel le (+)-néomenthol est fourni sous la forme d'un flux de néomenthol comprenant du (+)-néomenthol et au moins l'un parmi le (+)-néoisomenthol, le (-)-isopulégol et le (-)-menthol.

3. Procédé selon la revendication 2, dans lequel le flux de néomenthol comprend du (+)-néomenthol en une quantité de 85 à 99 % en poids, sur la base du poids total du flux de néomenthol.

4. Procédé selon l'une quelconque des revendications précédentes, dans lequel, pendant l'ajout de la solution aqueuse de peroxyde d'hydrogène, une température du mélange de réaction dans la plage de 55 à 90 °C est maintenue.

5. Procédé selon l'une quelconque des revendications précédentes, dans lequel le catalyseur à base de tungstène (VI) est un catalyseur à base d'orthotungstate.

6. Procédé selon la revendication 5, dans lequel le catalyseur à base de tungstène (VI) est le tungstate de sodium ou un hydrate de celui-ci.

7. Procédé selon l'une quelconque des revendications précédentes, dans lequel le catalyseur de transfert de phase est un composé de formule (I)
R₄N⁺X⁻ (I)
dans lequel chaque R est indépendamment choisi parmi alkyle en C₁-C₁₈, aryle en C₆-C₁₈, hétéroaryle en C₅-C₁₈ et (aryle en C₆-C₁₈)-(alkyle en C₁-C₁₀) ; et X⁻ est un anion.

8. Procédé selon la revendication 7, dans lequel le premier mélange comprend un composé de formule I, dans lequel X⁻ est un anion halogénure, et un acide fort, en particulier l'acide sulfurique.

9. Procédé selon la revendication 8, dans lequel le composé de formule (I) est le chlorure de méthyltrioctylammonium.

10. Procédé selon la revendication 7, dans lequel le catalyseur de transfert de phase contenu dans le premier mélange est un sel d'ammonium quaternaire de formule (Ib)
R₄N⁺HSO₄⁻ (Ib),
dans lequel chaque R est indépendamment choisi parmi alkyle en C₁-C₁₈, aryle en C₆-C₁₈, hétéroaryle en C₅-C₁₈ et (aryle en C₆-C₁₈)-(alkyle en C₁-C₁₀).

11. Procédé selon l'une quelconque des revendications précédentes, dans lequel le premier mélange et le deuxième mélange sont pratiquement exempts de solvant organique.

12. Procédé selon l'une quelconque des revendications précédentes, dans lequel de l'eau est ajoutée en une quantité suffisante de sorte qu'une phase aqueuse et une phase organique sont formées pendant le traitement aqueux.

13. Procédé selon la revendication 12, dans lequel le rapport en poids de l'eau ajoutée à la phase organique est d'au moins 1:1, en particulier dans lequel le rapport en poids de l'eau ajoutée à la phase organique est dans la plage de 1:1 à 3:1.

14. Procédé selon l'une quelconque des revendications précédentes, dans lequel le traitement aqueux comprend l'ajout d'une solution aqueuse d'un formiate de métal alcalin.
